# EUROPEAN PATENT APPLICATION

(11) **EP 3 888 540 A1**
(43) Date of publication of application: **06.10.2021**
(21) Application number: 20167515.4
(22) Date of filing: 01.04.2020
(51) Int. Cl.: A61B 5/0408, A61B 17/54, A61B 5/0478, A61B 5/0492

(54) **SKIN PREPARATION AND BIOPOTENTIAL MEASUREMENT DEVICE**

(71) Applicant: Koninklijke Philips N.V., 5656 AG Eindhoven (NL)
(72) Inventor: RABOTTI, Chiara, 5656 AE Eindhoven (NL); HAMELMANN, Paul Christoph, 5656 AE Eindhoven (NL); SIMONS, Evelyn Josefina Maria, 5656 AE Eindhoven (NL)
(74) Representative: Philips Intellectual Property & Standards

(57) **Abstract**

The invention provides a biopotential measurement device comprising a skin adhesion component for attachment to the skin, wherein the skin adhesion component comprises an aperture. A removable abrasive component is mounted in the aperture of the skin adhesion component. The removable abrasive component is for preparing the skin for biopotential measurements and is for subsequent removal from the aperture. An electrode is provided for mounting in the aperture of the skin adhesion component after the removal of the removable abrasive component. This provides a simple to use device with consistent performance.

## Description

### FIELD OF THE INVENTION

The invention relates to devices, systems and methods for biopotential measurements. In particular, the invention relates to a device, systems and methods for preparing the skin for biopotential measurements and subsequently performing the biopotential measurements.

### BACKGROUND OF THE INVENTION

The electric power system is a major source of interference in any situation of biopotential measurement, such as the electrocardiogram (ECG), the electromyogram (EMG) the electroencephalogram (EEG), and the electrohysterogram (EHG). The detrimental effect of these artifacts can be crucial for signals with a low signal-to-noise ratio (SNR), such as the fetal ECG recorded non-invasively from the mother's abdomen during pregnancy.

Besides providing power to the recording device itself, power lines are connected to other pieces of equipment and appliances in the room. There are also power lines in the walls, floor and ceiling running past the room to other points in the building. The source of this interference is therefore unavoidably present in any recording situation. Power lines can affect the biopotential recording and introduce interference at the line frequency (50 Hz or 60 HZ a.c. depending on the geographical region) in the recorded traces. Such interference appears on the recording as a result of two mechanisms, electric and magnetic, each operating singly or together.

The electric field coupling between a power line and the recording device or the patient is a result of the electric field surrounding a main power line and the power cords connecting different pieces of apparatus to the electric outlets. This field can be present even when the apparatus is turned off and, due to the capacitive coupling between the field and the system, the resulting currents flow through tissue and electrode impedances, thus producing alternating current (ac) potentials. These unwanted potentials appear at the output of the measurement chain mixed with the recorded biopotential and, depending on their amplitude, can irreversibly affect measurement and interpretation of the desired signal.

When recording biopotentials on the skin surface, the amplitude of the interference appearing at the output of an amplifier can be minimized by lowering the skin-electrode impedance, particularly at the location of a ground electrode, and making the electrode impedance of all recording electrodes as similar as possible. In general, measuring systems (amplifiers) with very high input impedance mitigate the effect of such interferences.

Additional sources of artifacts in biopotential measurements are due to electrode motion. Biopotentials are measured by (polarizable) electrodes, usually in contact with the skin through an electrolyte that allows a double layer of charge to form at the interface. If one of the measuring electrodes moves with respect to the electrolyte, the distribution of charges is mechanically disturbed. The electrode half-cell potential therefore changes together with the electrolyte-gel-skin potential, introducing a potential difference between the measuring electrodes, which is usually referred to as a motion artifact.

It has been demonstrated that artifacts can be significantly reduced when the stratum corneum (outer skin layer) is removed by mechanical abrasion with a fine abrasive paper or gel. This method also helps to reduce the epidermal component of the skin impedance. Therefore, skin preparation by removal of (part of) the stratum corneum can significantly improve rejection of artifacts. However, the removal of the body's outer protective barrier makes that region of the skin more susceptible to irritation from an electrolyte gel.

Accurate skin preparation to remove (part of) the stratum corneum is of key importance for reducing artifacts in biopotential measurements and improving signal-to-noise ratio. In current practice, prior to surface biopotential measurement, the skin is prepared manually using abrading pads (sand paper) or gel. Skin must be prepared at exactly the same position where the sensing part of the electrode(s) will be in contact with the skin. Some skin redness arising as a consequence of abrasion can help localizing the prepared area. However, current manual skin preparation does not facilitate the identification of the exact location of the previously prepared area prior to electrode positioning. Briefly, current manual methods for skin preparation prior to biopotential measurement are affected by the following disadvantages:
(i) Skin preparation and electrode placing are time consuming processes.
(ii) A different amount of stratum corneum may be removed at different electrode locations (there are typically at least two in any biopotential measurement).
(iii) Preparation is not homogeneous through the whole sensing surface.
(iv) It is very difficult to limit abrasion to the area in direct contact with the sensing surface in order to limit the extent of irritated skin.
(v) It can lead to excessive redness, indicating excessive skin removal increasing the risk of irritation.

The above mentioned disadvantages would be even more challenging to a non-experienced user, as it would be required that the user (e.g. pregnant women) would perform this skin preparation (e.g. women's abdomen).

Therefore, there is a need for a biopotential measurement device which can appropriately and consistently prepare the skin for biopotential measurements.

### SUMMARY OF THE INVENTION

The invention is defined by the claims.

According to examples in accordance with an aspect of the invention, there is provided a biopotential measurement device comprising:
a skin adhesion component for attachment to the skin, wherein the skin adhesion component comprises an aperture;
a removable abrasive component mounted in the aperture of the skin adhesion component, wherein the removable abrasive component is for preparing the skin for biopotential measurements and is for subsequent removal from the aperture; and
an electrode for mounting in the aperture of the skin adhesion component after the removal of the removable abrasive component.

The skin preparation device may be placed on the skin of a subject, wherein the electrode is for performing biopotential measurements. However, the outer-most layer of the skin (stratum corneum) has an increased impedance (compared to the lower layers of the epidermis) and if not properly and evenly removed increases the effect of motion artefacts and external interference from electric and magnetic fields. Thus, the removable abrasive component prepares the skin by removing part (or all) of the stratum corneum from the desired area, such that the skin-electrode contact is significantly improved.

Typical methods to improve the skin-electrode contact include piercing the skin or the use of manual abrasive pads. Piercing the skin may be painful to the subject and manual abrasive pads may be time consuming and remove a different amount of stratum corneum each time. The removable abrasive component ensures that the skin is prepared consistently with other areas of skin (which have other electrodes attached), and for measurements at different times. Additionally, the area of skin which is prepared can be minimized to the area of skin which is in contact with the electrode.

The removable abrasive component may comprise stripping tape.

The removable abrasive component may comprise an abrasive gel or sandpaper.

The removable abrasive component may further comprise a carrier having a thread and wherein the aperture of the skin abrasive component is threaded. The abrasive component may be configured for removal by unscrewing from the aperture of the skin adhesion component.

The electrode may comprise:
a sensing surface for biopotential measurements; and
an attachment surface for attachment to the skin adhesion component after the removal of the removable abrasive component.

A removable cover may be mounted between the skin adhesion component and the attachment surface of the electrode, wherein the removable cover is mechanically attached to the attachment surface of the electrode.

The removable cover may be mechanically attached to the attachment surface of the electrode with:
adhesive material;
one or more sliding clips; or
one or more fixing clips.

The removable cover may further comprise a tab for assisting manual removal from the skin adhesive component.

The abrasive component may be attached to the removable cover.

The invention also provides a system for analyzing biopotential measurements, the system comprising:
two or more biopotential measurement devices; and
a processor electrically connected to the biopotential measurement devices, wherein the processor is configured to:
   receive data from electrodes of the two or more biopotential measurement devices; and
   analyze the data.

For electrophysiological measurements, at least two electrodes at two locations are typically needed. However, if the skin preparation is not the same (i.e. the same amount of stratum corneum removed) at all locations of the electrodes, the skin impedance will be different at different electrodes thus reducing the accuracy of the measurements (e.g. by reducing signal to noise ratio).

By using two or more biopotential measurement devices with the same abrasive component, homogeneous skin preparation can be ensured for all of the electrodes. This further allows less experienced operators to perform biopotential measurements.

The processor may be electrically connected to the one or more devices with one of:
one or more wires; or
a wireless connection.

The invention also provides a method for preparing skin for biopotential measurements, the method comprising:
attaching a skin adhesion component to the skin, wherein the skin adhesion component comprises an aperture and a removable abrasive component in the aperture of the skin adhesion component;
removing the removable abrasive component from the aperture of the skin adhesion component; and
attaching an electrode in the aperture of the skin adhesion component for biopotential measurements.

Removing the removable abrasive component may comprise unscrewing the removable abrasive component from the aperture of the skin adhesion component by rotation.

The method may further comprise removing a removable cover from the skin adhesion component thereby to expose the removal abrasive component.

Removing the removable cover may also remove the removable abrasive component.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows the cross section of a first example of a biopotential measurement device;
Figure 2 shows the cross section of a second example of a biopotential measurement device based on tape stripping;
Figure 3 shows a first example of the steps performed by a biopotential measurement device in order to perform the biopotential measurements;
Figure 4 shows the cross section of a third example of a biopotential measurement device based on rotational abrasion;
Figure 5 shows a second example of the steps performed by a biopotential measurement device in order to perform the biopotential measurements;
Figure 6 shows an example of a system for performing biopotential measurements; and
Figure 7 shows a flow diagram for a method for preparing skin for biopotential measurements.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a biopotential measurement device comprising a skin adhesion component for attachment to the skin, wherein the skin adhesion component comprises an aperture. A removable abrasive component is mounted in the aperture of the skin adhesion component. The removable abrasive component is for preparing the skin for biopotential measurements and is for subsequent removal from the aperture. An electrode is provided for mounting in the aperture of the skin adhesion component after the removal of the removable abrasive component. This provides a simple to use device with consistent performance.

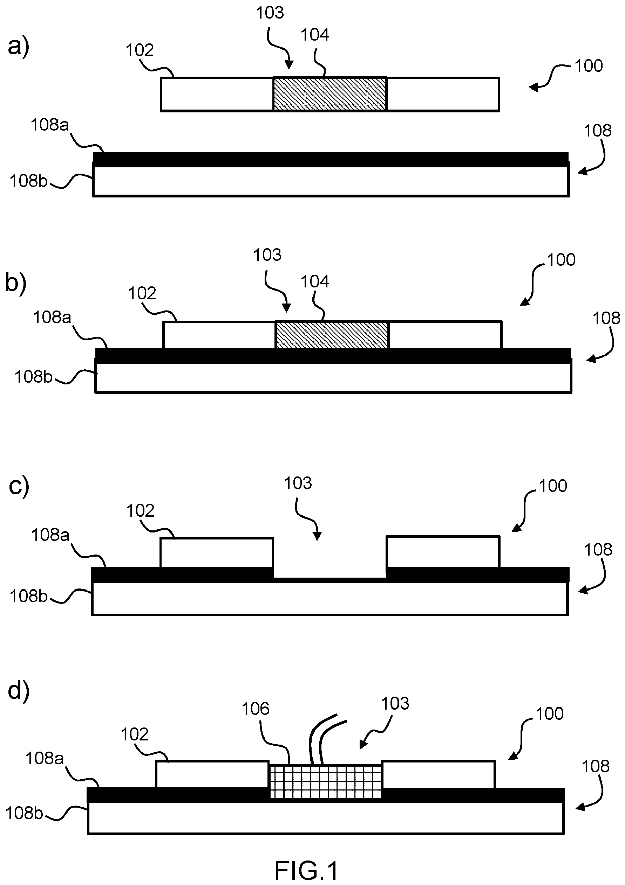

Figure 1 shows the cross section of a first example of a biopotential measurement device 100, at different stages of its use. The biopotential measurement device 100 integrates a skin adhesion component 102, a removable abrasive component 104 for removing the stratum corneum and an electrode 106 for electrophysiological measurements into a low-cost single-use disposable device.

The skin adhesion component 102 (i.e. adhesive mask) can be positioned at the desired skin location from which the electrophysiological signals are obtained.

The removable abrasive component 104 of the device 100 enables well-defined removal of the stratum corneum 108a of the skin 108 at the site predetermined by the adhesive mask 102.

The abrasive component 104 can be removed to allow the electrode 106 to be pushed into the aperture 103 of the adhesive mask 102.

Figure 1 a) shows the device 100 before it is placed on the desired skin location. Placement of electrodes 106 is very important during ECG measurements, as the correct placement increases the signal to noise ratio.

Figure 1 b) shows the device 100 when placed on the skin 108. The top layer of the skin 108 is the stratum corneum 108a, and this layer of the skin increases the impedance between an electrode 106 and the skin 108. The device 100 is placed on the area of the desired area of the skin for the biopotential measurements.

Figure 1 c) shows the device 100 after the abrasive component 104 has been removed, thus leaving an aperture 103. The abrasive component can be configured such that, when it is removed, it accurately and consistently removes an amount of the stratum corneum 108a. Thus, the impedance between the skin 108 (and the lower layer of the skin, the dermis 108b) and an electrode 106 are reduced. The device 100 ensures only a minimum amount of stratum corneum 108a is removed necessary for the placement of an electrode 106, thus reducing overall irritation.

Figure 1 d) shows the device after the abrasive component 104 has been removed and an electrode 106 has been placed in the aperture 103. The electrode 106 may now perform biopotential measurements on the skin 108.

The combination of this integrated device 100 and application method ensure reduced time of preparation, and therefore improved work-flow, homogeneous skin preparation below the sensing surface and among the different electrodes, limit abrasion to the desired surface, and avoid excessive skin redness. Finally, by easing the skin-preparation phase and electrode placement, the device 100 makes accurate biopotential measurement possible also for less experienced operators (e.g. pregnant women performing biopotential measurements at home).

The method for skin preparation, and hence the abrasive component 104 of the device 100, may differ depending on the use case, user preference, manufacturing and other factors. For example, the stratum corneum 108a may be removed by tape stripping. Tape stripping is currently used for removing the stratum corneum 108a prior to tissue testing. The removal of the whole stratum corneum 108a requires repeated tape stripping. However, a single tape stripping action is typically sufficient to remove the necessary amount of stratum corneum 108a without causing skin irritation. The stratum corneum 108a may also be removed by abrasion, operated by an embedded rotating pad. This guarantees a well-defined skin preparation.

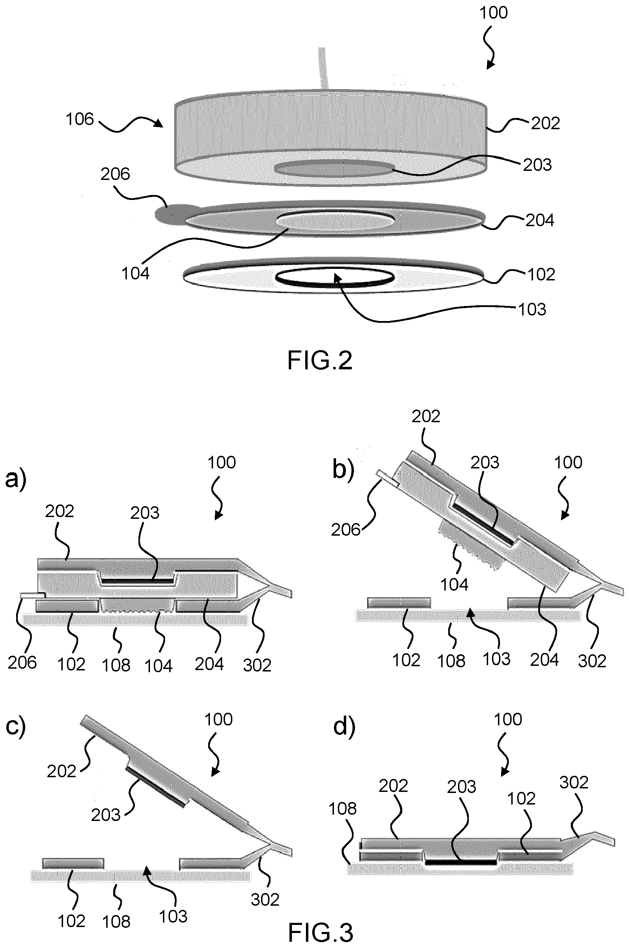

Figure 2 shows the cross section of a second example of a biopotential measurement device based on tape stripping. In this example, the adhesive mask 102 has a hole 103 in correspondence to the desired location for the electrode 106 (sensing surface).

There is a removable cover 204 which has dedicated tape-stripping material 104 (an abrasive component) in alignment with the hole 103. The removable cover 204 also has a tab 206 for assisting in the removal of the removable cover. The electrode 106 comprises an attachment surface 202 and a sensing surface 203. The sensing surface 203 thus performs the biopotential measurements. The components may be kept together mechanically in order to allow placement and execution of skin preparation.

Figure 3 shows a first example of the steps performed when using a biopotential measurement device of the type shown in Figure 2 in order to perform the biopotential measurements.

Figure 3 a) shows the device 100 placed on the skin 108. The removable cover is attached to the abrasive component 104 and to the attachment surface 202. There is also a hinge 302 between the adhesive mask 102 and the attachment surface 202.

Figure 3 b) shows the device 100 when abrasive component 104 has been removed. In this example, the abrasive component 104 is attached to the removable cover 204, such that when the tab 206 is pulled and the device is "opened", the skin 108 becomes prepared for biopotential measurements. The removable cover 204 is attached to the attachment surface 202 by, for example, adhesive material, sliding slips or fixing clips.

Figure 3 c) shows the device 100 after the removable cover 204, and thus the adhesive component 204, is removed. The skin 108 is now prepared from the biopotential measurements.

Figure 3 d) shows the device 100 after is has been "closed". The sensing surface 203 is now in contact with the prepared skin and the hinge 302 and the attachment surface 202 (which is now mechanically attached to the adhesive mask 102) ensures that the sensing surface 203 does not lose contact with the skin.

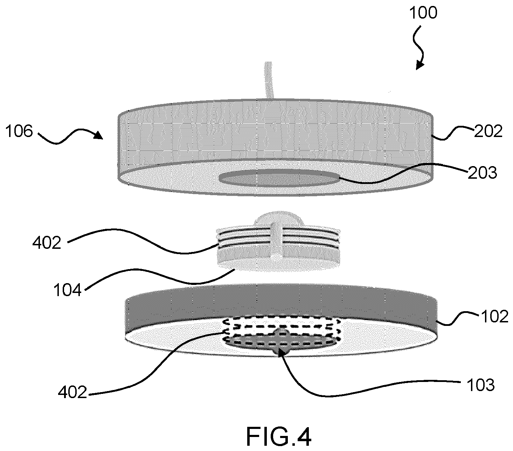

Figure 4 shows the cross section of a third example of a biopotential measurement device 100 based on rotational abrasion. In this example, the adhesive mask 102 has an aperture 103 with threads 402 and the abrasive component 104 also has threads 402, such that removing the abrasive component 104 from the adhesive mask 102 involves unscrewing the abrasive component 104 from the aperture 103. The rotational abrasion may be caused by, for example, sandpaper, abrasive gel or abrasive material on the abrasive component 104.

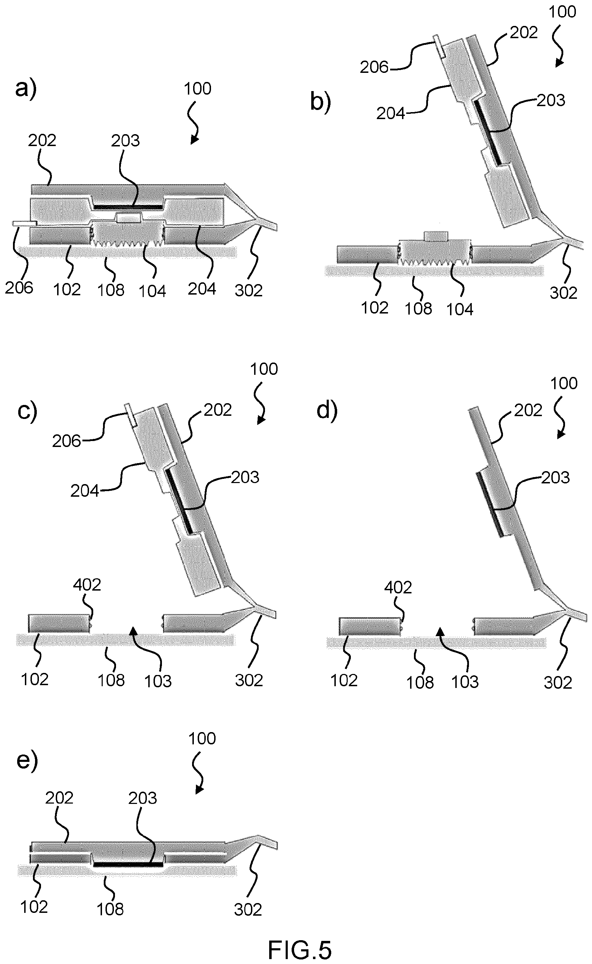

Figure 5 shows a second example of the steps performed when using a biopotential measurement device of the type shown in Figure 4 in order to perform the biopotential measurements. In this example, both the adhesive mask 102 and the removable cover 204 have an aperture 103 in the location corresponding to the sensing surface 203. The adhesive mask 102 has a thread 402 within the wall of the aperture that allows screwing a support with the abrasive component 104. The support can only be removed after a predefined (by design) number of turns. This can be done by a dedicated thread pattern design.

Figure 5 a) shows the device 100 placed on the skin 108.

Figure 5 b) shows the device 100 when it has been "opened". In this example, the abrasive component 104 is not attached to the removable cover 204. In order to prepare the skin 108, the abrasive component 104 is unscrewed from the adhesive mask 102.

Figure 5 c) shows the device 100 after the abrasive component 104 has been removed and the skin 108 is prepared for biopotential measurements.

Figure 5 d) shows the device 100 after the removable cover 204 has been removed, thus exposing the sensing surface 203.

Figure 5 e) shows the device 100 after is has been "closed". The sensing surface 203 is now in contact with the prepared skin and the hinge 302 and the attachment surface 202 (which is now mechanically attached to the adhesive mask 102) ensure that the sensing surface 203 does not loose contact with the skin.

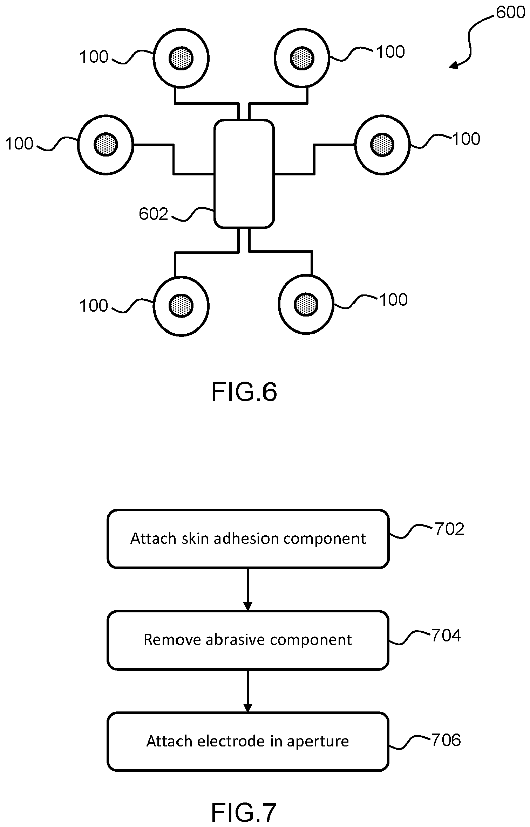

Figure 6 shows an example of a system 600 for performing biopotential measurements. In this example, the system 600 comprises six devices 100 for performing the biopotential measurements, however, the number of devices 100 may change depending on the placement of the devices 100, the area of the body for which the measurements are for, the judgement of the user performing the measurements and other factors.

The system 600 also comprises a processor 602 for analyzing the biopotential data from the devices 100. In order to achieve good signal to noise ratios, and thus perform accurate measurements, the impedance between the skin 108 and the electrodes 106 of the devices 100 must be the same (or as similar as possible). The biopotential measurement devices 100 previously described ensure the same level of skin preparation, thus making the impedance between the electrodes 106 and the skin 108 similar throughout all of the devices 100.

Figure 7 shows a flow diagram for a method for preparing skin for biopotential measurements. A skin adhesion component is attached to the skin in step 702, wherein the skin adhesion component comprises an aperture and a removable abrasive component in the aperture of the skin adhesion component. The removable abrasive component is then removed from the aperture of the skin adhesion component, in step 704, and an electrode is subsequently attached to the skin through the aperture of the skin adhesion component for biopotential measurements, in step 706.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

If the term "adapted to" is used in the claims or description, it is noted the term "adapted to" is intended to be equivalent to the term "configured to".

Any reference signs in the claims should not be construed as limiting the scope.

## Claims

1. A biopotential measurement device (100) comprising:
a skin adhesion component (102) for attachment to the skin (108), wherein the skin adhesion component (102) comprises an aperture (103);
a removable abrasive component (104) mounted within the aperture (103) of the skin adhesion component (102), wherein the removable abrasive component (104) is for preparing the skin (108) for biopotential measurements and is for subsequent removal from the aperture (103); and
an electrode (106) for mounting within the aperture (103) of the skin adhesion component (102) after the removal of the removable abrasive component (104).

2. The device (100) of claim 1, wherein the removable abrasive component (104) comprises a stripping tape.

3. The device (100) of claim 1, wherein the removable abrasive component (104) comprises an abrasive gel or sandpaper.

4. The device (100) of claim 3, wherein the removable abrasive component (104) comprises a carrier having a thread (402) and the aperture (103) of the skin adhesion component (102) is threaded, and wherein the removable abrasive component (104) is configured for removal by unscrewing from the aperture (103) of the skin adhesion component (102).

5. The device (100) of any one of claim 1 to 4, wherein the electrode (106) comprises:
a sensing surface (203) for biopotential measurements; and
an attachment surface (202) for attachment to the skin adhesion component (102) after the removal of the removable abrasive component (104).

6. The device (100) of claim 5, further comprising a removable cover (204) mounted between the skin adhesion component (102) and the attachment surface (202) of the electrode (106), wherein the removable cover (204) is mechanically attached to the attachment surface (202) of the electrode (106).

7. The device (100) of claim 6, wherein the removable cover (204) is mechanically attached to the attachment surface (202) of the electrode (106) with:
adhesive material;
one or more sliding clips; or
one or more fixing clips.

8. The device (100) of any one of claims 6 or 7, wherein the removable cover (204) further comprises a tab (206) for assisting manual removal.

9. The device of any one of claims 6 to 8, wherein the removable abrasive component (104) is attached to the removable cover (204).

10. A system (600) for analyzing biopotential measurements, the system (600) comprising:
two or more biopotential measurement devices (100) as claimed in any one of claims 1 to 9; and
a processor (602) electrically connected to the biopotential measurement devices (100), wherein the processor (602) is configured to:
receive data from electrodes (106) of the two or more biopotential measurement devices (100); and
analyze the data.

11. The system (600) of claim 10, wherein the processor (602) is electrically connected to the one or more devices (100) with one of:
one or more wires; or
a wireless connection.

12. A method for preparing skin for biopotential measurements, the method comprising:
attaching a skin adhesion component to the skin (702), wherein the skin adhesion component comprises an aperture and a removable abrasive component in the aperture of the skin adhesion component;
removing the removable abrasive component from the aperture of the skin adhesion component (704); and
attaching an electrode in the aperture of the skin adhesion component for biopotential measurements (706).

13. The method of claim 12, wherein removing the removable abrasive component comprises unscrewing the removable abrasive component from the aperture of the skin adhesion component by rotation.

14. The method as claimed in any one of claim 12 or 13, further comprising removing a removable cover from the skin adhesion component thereby to expose the removal abrasive component.

15. The method of claim 14, wherein removing the removable cover also removes the removable abrasive component.
